# EUROPÄISCHE PATENTSCHRIFT

(11) **EP 0 747 079 B1**
(45) Veröffentlichungstag und Bekanntmachung des Hinweises auf die Patenterteilung: **08.03.2000**
(21) Anmeldenummer: 96108980.2
(22) Anmeldetag: 05.06.1996
(51) Int. Cl.: A61M 21/00, A61H 1/00

(54) **Entspannungseinrichtung**
Relaxation device
Dispositif de relaxation

(30) Priorität: 06.06.1995 DE 19520064
(43) Veröffentlichungstag der Anmeldung: 11.12.1996
(73) Patentinhaber: ENKO GmbH & Co., 41199 Mönchengladbach (DE)
(72) Erfinder: Clausen, Günter R., Dr., 41470 Neuss (DE); König, Klaus, 41464 Neuss (DE); Rödelbronn, Horst, 41469 Neuss (DE); Steuer, Rudolf, 4950 Ovifat-Robertville (BE)
(74) Vertreter: Bonsmann, Manfred, Dipl.-Ing.

(56) Entgegenhaltungen:
- DE-A- 2 552 488
- NL-A- 7 702 649
- DATABASE WPI Section EI, Week 8719 Derwent Publications Ltd., London, GB; Class S05, AN 87-134383 XP002009886 & SU-A-1 258 420 (A MED WORK HYGIENE) , 23.September 1986

## Beschreibung

Die Erfindung bezieht sich auf eine Entspannungseinrichtung mit einer Vibrationseinrichtung und einer daran angeschlossenen, in Vibrationen versetzbaren Matratze, welche einen aufblasbaren Körper aufweist, bei dem wenigstens die Liegefläche aus einem flexiblen Material hergestellt ist, wobei die Vibrationseinrichtung eine zur periodischen Veränderung des Innendrucks des aufblasbaren Körpers vorgesehene Steuereinrichtung aufweist.

Eine derartige Entspannungseinrichtung ist aus NL-A-7 702 649 bekannt.

Viele Patienten leiden an nervösen, durch die Unruhen des Alltages bedingten Beschwerden. Auf Grund ihres gestörten Nervensystems sind solche Patienten auch kaum in der Lage, sich von diesen Beschwerden durch regelmäßige körperliche, geistige und seelische Entspannung zu befreien. Die Folge hiervon sind im körperlichen, geistigen oder seelischem Bereich angesiedelte Beschwerden wie Kopfschmerzen, Schlafstörungen, Neuralgien, Konzentrationsschwächen, Übermüdungszustände, Situationsängste, Lampenfieber, Depressionen oder Angstneurosen, nur um einige dieser Beschwerden zu nennen.

Linderung und Wiederherstellung des Normalzustandes kann bei diesen Patienten durch eine regelmäßige Entspannung erreicht werden. Zur Herbeiführung eines totalen Entspannugszustands sind verschiedene Verfahren wie Yoga und autogenes Training bekannt. Die Wirksamkeit derartiger Verfahren setzt jedoch voraus, daß der betroffene Patient trotz der ständigen Reizüberflutung noch die Kraft, den Willen und die Fähigkeit hat aus eigenem Antrieb, also aktiv, über einen längeren Zeitraum in einen Zustand der inneren Ruhe und Entspannung zu kommen.

Es ist bereits versucht worden, Hilfsmittel zu schaffen, die dem Patienten das Entspannen erleichtern sollen. Ein bekanntes derartiges Hilfsmittel besteht aus einer aufblasbaren Luftmatratze, die an eine Vibrationseinrichtung angeschlossen ist, welche mittels eines Blasebalges - immer mit der gleichen Luftmenge - pneumatische Vibrationen ausführt. Diese Vorrichtung ist mit einer von Hand bedienbaren Steuervorrichtung versehen.

Entspannungseinrichtungen mit derartigen Vibrationseinrichtungen haben sich jedoch als wenig brauchbar erwiesen, weil sie sich - bezogen auf die Atemamplitude - nicht ohne Unterbrechung der Entspannungsphase an die individuellen Verhaltensweisen der Patienten beim Entspannungsversuch anpassen lassen. Für ein solche Anpassung ist es nämlich erforderlich, stets zuerst die Vibrationseinrichtung anzuhalten und das Gehäuse zu öffnen, um die Koppel, die bei der bekannten Vorrichtung eine Kupplungsverbindung des Blasebalg mit der Kurbelscheibe am Motor herstellt, in der Exentrizität auf der Kurbelscheibe zu verstellen, um einen größeren oder kleineren Hub zu erreichen. Weiterhin wird bei der bekannten Einrichtung die postexspiratorische Phase nicht berücksichtigt. Weiterhin muß entweder der Patient selbst oder eine weitere Person die Atemfrequenz (bzw. die Hubgeschwindigkeit des Blasebalges) steuern.

Der Erfindung liegt die Aufgabe zugrunde, eine Entspannungseinrichtung zu schaffen, die sich vollautomatisch der Atemfrequenz, der Atemamplitude und der postexspiratorischen Phase des Patienten anpaßt und diese Größen dann - bis zur vollständigen Entspannung des Patienten nach therapeutischmedizinischen Gesichtspunkten - ohne Einwirkung des Patienten oder einer anderen Person verändert.

Eine weitere Aufgabe besteht darin, daß sich diese Entspannungseinrichtung, falls erforderlich, in der Atemfrequenz auch manuell durch einen Therapeuten steuern läßt. Auch hierbei soll sich die Atemamplitude und die postexspiratorische Phase vollautomatisch der Atemfrequenz des Patienten anpassen.

Erfindungsgemäß wird dies bei einer Entspannungseinrichtung der eingangs genannten Art dadurch erreicht, daß eine Sensoreinrichtung zur Erfassung der Atemfrequenz eines auf der Liegefläche liegenden Patienten vorgesehen ist, und mittels der Steuereinrichtung der Aufwärtshub der Liegefläche dem Einatmungsvorgang und der Abwärtshub der Liegefläche dem Ausatmungsvorgang und die postexspiratorische Phase einem Hubstillstand anpaßbar ist, und der Steuereinrichtung eine Tabellenspeichereinrichtung mit einer die durchschnittliche relative Dauer des Einatmungsvorganges, des Ausatmungsvorganges und der postexspiratorischen Ruhephase bei unterschiedlichen Atemfrequenzen enthaltenden Tabelle zugeordnet ist, und die Hubfrequenz entsprechend den der jeweils gemessenen Atemfrequenz des Patienten oder eingestellten Atemfrequenz entsprechenden gespeicherten Tabellenwerten ausgeführt wird, und der Steuereinrichtung eine Folgesteuereinrichtung zugeordnet ist, durch deren Wirkung die Hubfrequenz der Vibrationseinrichtung in bestimmten Zeitabständen stufenweise herabsetzbar ist, und eine Vergleichseinrichtung vorgesehen ist, über die u. a. feststellbar ist, ob eine bestimmte Zeit nach dem Herabsetzen der Hubfrequenz eine Anpassung der tatsächlichen Atemfrequenz des Patienten an die herabgesetzte Hubfrequenz erfolgt ist.

Die Luftbewegungseinrichtung kann in einer Einstellung "auto" durch die Atemfrequenz des Patienten gesteuert und in einer Einstellung "manuell" an die Atemfrequenz durch einen Therapeuten angepaßt und verändert werden. In beiden Fällen wird die Atemamplitude und die postexspiratorische Phase automatisch, nach einem vorgegebenen Schema, an die jeweilige Atemfrequenz anpaßt.

Die Erfindung geht davon aus, daß eine totale Entspannung und eine tiefe und ruhige Atmung nur erreichbar sind, wenn der Patient nicht aktiv in die Steuerung eingreifen muß, und daß viele Patienten nicht in einen entspannten Zustand gelangen können, wenn eine andere Person zugegen ist, um die Steuerung zu bedienen, und daß bei einigen Patienten der Therapeut zugegen sein muß und dieser dann in der Lage sein soll, die Geschwindigkeit der Atemfrequenz manuell zu verändern.

Nach einer vorteilhaften Ausgestaltung der Erfindung ist vorgesehen, daß die Sensoreinrichtung einen Atemsensor aufweist, und über diesen und/oder Druckschlauch die Atemfrequenz, die Atemamplitude und die postexspiratorische Phase am Körper des Patienten zu messen und diese Bewegungsmessung an einen Computer zur Verarbeitung weiterzuleiten.

Vorteilhafterweise ist der Computer so programmiert, daß er über einen kurzen Zeitraum die Atemfrequenzen mißt, diese in sein Programm einordnet, mittelt und dann mit dieser gemittelten Atemfrequenz die angeschlossene Luftmatratze über ein durch den Computer steuerbares Luftventil so in Bewegung versetzt, daß diese in ihrer Bewegung, d. h. in der Geschwindigkeit (Atemfrequenz) und vorzugsweise auch in der Tiefe (Atemamplitude), und in der Nachbeatmungspause (postexspiratorische Phase) genau der Atemfrequenz des Patienten entspricht.

Nach einer exakt festgelegten Zeit verringert dann der Computer die Bewegung der Luftmatratze um eine Atembewegung pro Minute. Dabei wird sowohl die Amplitude als auch die postexspiratorische Phase automatisch verändert, und zwar entsprechend den Werten eines Tabellenspeichers, welcher die durchschnittlichen Verhältniswerte der einzelnen Phasen des Atmungsvorganges bei unterschiedlichen Anzahlen von Atemhüben pro Minute enthält. Diese Verringerung der Bewegung setzt sich nach einem festgelegten Rhythmus so lange fort, bis der Patient in einem tiefen Entspannungszustand nur noch sechs Atemhübe pro Minute macht.

In dem Computerprogramm ist vorteilhafterweise berücksichtigt, daß nicht jeder Patient in jeder Situation die vorgegebene Verringerung der Bewegung annimmt. Es ist vorgesehen, daß dem Patienten in einem bestimmten Takt immer wieder eine Verringerung der Atemfrequenz angeboten wird. Folgt er ihr, wird das Programm die Atemfrequenzen pro Minute so, wie in der zuvor beschriebenen Weise verringern, wie es dem Patienten angemessen ist, aber niemals unter sechs Bewegungen pro Minute.

Weiterhin kann vorgesehen sein, daß bei einem plötzlichen Hochschnellen der Atemfrequenz des Patienten, spätestens nach sechs gemessenen Atemfrequenzen, der Computer die Luftmatratze in die gemessene, erhöhte Bewegungsfrequenz versetzt, und dann wieder - wie zuvor beschrieben - versucht, dem Patienten eine Verringerung der Atemfrequenz anzubieten.

Ebenfalls kann vorgesehen sein, daß bei einer schnelleren Beruhigung des Patienten, als es die Programmschritte vorsehen - spätestens nach sechs gemessenen Atemfrequenzen - der Luftmatratze die gemessene, erniedrigte Bewegungsfrequenz aufgegeben wird. Danach wird - wie zuvor beschrieben - der Computer versuchen, die Atemfrequenz weiter bis auf sechs Bewegungen pro Minute zu verringern.

Die Luftbewegungseinrichtung besteht aus einem äußeren Gehäuse aus Aluminium, das mit Antidröhnmatten ausgekleidet ist. Dieses äußere Gehäuse ist mittels Gummischwinglagern mit dem inneren Gehäuse verbunden.

Das innere Gehäuse besteht aus einem schwingungsneutralen Material, welches die Tonschwingungen, die von einem Hochdruck-Lüftermotor, einem Servomotor und dem aus dem Steuerventil austretenden Luftstrom erzeugt werden, nicht verstärkt. Zusätzlich ist dieses Gehäuse mit Schallschluckmatten ausgekleidet.

In einer oberen Kammer des inneren Gehäuses ist ein Hochdruck-Lüftermotor installiert, der permanent in der Einschaltzeit der Bewegungseinrichtung Raumluft ansaugt und in Richtung Luftmatratze ausbläst.

Der Kammer für den Hochdruck-Lüftermotor ist eine weitere Kammer vorgeschaltet, in der mittels einer Klappe die Ansaugluftmenge manuell steuerbar ist. Diese manuelle Steuerung erfolgt bei der Ersteinstellung der Einrichtung.

Der Luftstrom in Richtung Luftmatratze wird mittels eines Ventils, das durch einen Servo-, Schritt- oder anderen in der Dreh- oder Schubrichtung steuerbaren Motor verstellbar ist, in vielfältiger Weise gesteuert. Dieses Ventil und der Steuermotor sitzen in einer Kammer im unteren Teil des inneren Gehäuses.

Dieser Kammer ist eine weitere Kammer nachgeschaltet, in der mittels einer Klappe die Auslaßluftmenge manuell steuerbar ist. Diese Steuerung erfolgt im Rahmen einer Grundeinstellung zum Ausgleich von Fertigungstoleranzen.

Der Hochdruck-Lüftermotor und das Ventil mit dem Steuermotor sind aus zwei Gründen in verschiedenen Kammern untergebracht, um nämlich zum einen die Ansaugluftmenge und die Auslaßluftmenge getrennt steuern zu können, und zum anderen deshalb, damit die aus dem Ventil austretende und nach außen abzuleitende überschüssige Luft keine Verwirbelungen in der Ansaugluft hervorrufen kann, was zu unerwünschten Flatterbewegungen der Matratze führen könnte.

Zunächst läßt das Ventil Luft in die Matratze, und zwar bis zu einem vorwählbaren Innendruck von 70 mm bis 120 mm Wassersäule. Nach Erreichen des Innendruckes wird der Luftstrom vom Ventil so verteilt, daß nur noch soviel Luft in die Matratze geführt wird, um den vorgewählten Innendruck zu erhalten. Die überschüssige Luft wird nach außen abgeleitet.

Der vorgewählte Innendruck wird auch durch entsprechend gesteuerte Ventileinstellungen beibehalten, wenn sich der Patient auf die Luftmatratze legt oder sich von ihr erhebt.

Selbstverständlich ist es während der Behandlung möglich, den Innendruck innerhalb der zuvor genannten Bandbreite auf den Druck einzustellen, den der Patient als angenehm empfindet.

Durch Öffnen und Schließen und Halten des Öffnungs- und Schließwinkels des Ventils wird die Luft in Richtung Luftmatratze so gesteuert, daß sie diese in der Atemfrequenz, der Atemamplitude und der postexspiratorischen Phase angepaßt in Bewegung versetzt wird und gleichzeitig den gemittelten Wert des Innendruckes (z.B. 100 mm Wassersäule) während der Auf- und Abbewegung beibehält.

In den Zeichnungen ist schematisch ein Ausführungsbeispiel der Erfindung dargestellt. Es zeigen:
- Fig. 1: eine schematische Darstellung der Gesamtvorrichtung;
- Fig. 2: eine Vorderansicht der Steuereinrichtung, teilweise in Schnittdarstellung;
- Fig. 3: eine Seitenansicht der Vorrichtung gemäß Fig. 2, teilweise in Schnittdarstellung.

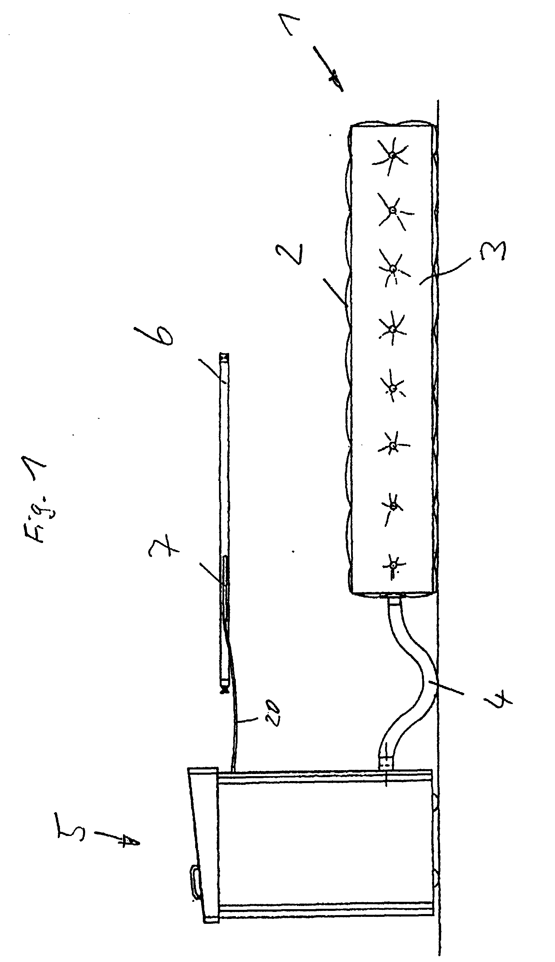

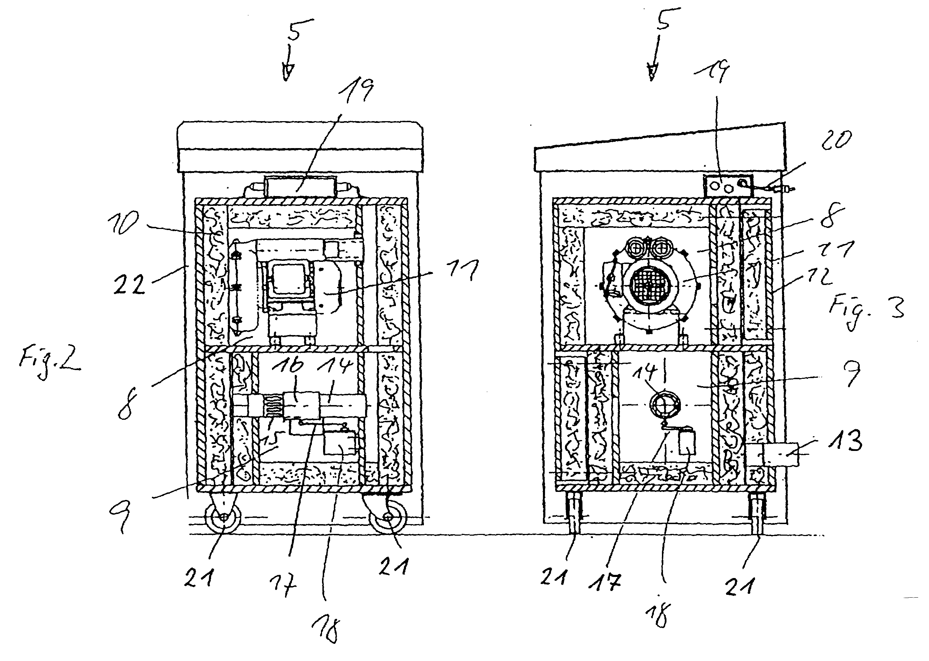

Eine schematisch dargestellte Matratze 1 weist eine Liegefläche 2 aus einem flexiblen Material auf. Unterhalb der Liegefläche ist ein Hohlraum 3 vorgesehen, welcher über einen Schlauch 4 mit einer insgesamt mit 5 bezeichneten Steuereinrichtung in Verbindung steht. Bei einem auf der Matratze 1 liegenden Patienten (nicht dargestellt) kann die Atemfrequenz mittels eines Brustgurtes 6 gemessen werden, welcher einen Atemsensor 7 aufweist. Die Meßwerte können der Steuereinrichtung 5 über eine Leitung 20 zugeleitet werden.

Die Steuereinrichtung 5 weist in einem beispielsweise aus dem Werkstoff Aluminium hergestellten, auf Rädern 21 fahrbaren Gehäuse 22 eine erste Kammer 8 und eine zweite Kammer 9 auf. Die Kammern sind mittels Schallschluckplatten ausgekleidet, von denen lediglich eine Schallschluckplatte das Bezugszeichen 10 trägt. In der Kammer 8 ist ein Hochdruck-Lüfter 11 angeordnet, welchem Luft über eine Lufteinlaßklappe 12 zuführbar ist. Ein Anschluß zur Anbringung des Schlauches 4 trägt die Bezugsziffer 13. Die Ausgangsseite des Hochdruck-Lüfters 11 steht mit der Innenseite eines Rohres 14 mit schlitzförmigen Luftaustrittsöffnungen 15 in Verbindung. Die Menge der austretenden Luft kann durch Verstellung eines Schiebers 16 eingestellt werden, der über eine Koppel 17 und einen Stellmotor 18 verstellbar ist.

Weiterhin weist die Steuereinrichtung einen Mikrocomputer 19 auf, welcher den Tabellenspeicher enthält und über den die Steuerung der Entspannungseinrichtung in der beschriebenen Weise erfolgt. Signale des Atemsensors 7 werden dem Mikrocomputer über die Leitung 20 zugeführt.

## Patentansprüche

1. Entspannungseinrichtung mit einer Vibrationseinrichtung und einer daran angeschlossenen, in Vibrationen versetzbaren Matratze (1), welche einen aufblasbaren Körper aufweist, bei dem wenigstens die Liegefläche (2) aus einem flexiblen Material hergestellt ist, wobei die Vibrationseinrichtung eine zur periodischen Veränderung des Innendrucks des aufblasbaren Körpers vorgesehene Steuereinrichtung (5) aufweist,
dadurch gekennzeichnet, daß
eine Sensoreinrichtung (7) zur Erfassung der Atemfrequenz eines auf der Liegefläche liegenden Patienten vorgesehen ist, und mittels der Steuereinrichtung der Aufwärtshub der Liegefläche dem Einatmungsvorgang und der Abwärtshub der Liegefläche dem Ausatmungsvorgang und die postexspiratorische Phase einem Hubstillstand anpaßbar ist, und der Steuereinrichtung eine Tabellenspeichereinrichtung mit einer die durchschnittliche relative Dauer des Einatmungsvorganges, des Ausatmungsvorganges und der postexspiratorischen Ruhephase bei unterschiedlichen Atemfrequenzen enthaltenden Tabelle zugeordnet ist, und die Hubfrequenz entsprechend den der jeweils gemessenen Atemfrequenz des Patienten oder eingestellten Atemfrequenz entsprechenden gespeicherten Tabellenwerten ausgeführt wird, und der Steuereinrichtung eine Folgesteuereinrichtung zugeordnet ist, durch deren Wirkung die Hubfrequenz der Vibrationseinrichtung in bestimmten Zeitabständen stufenweise herabsetzbar ist, und eine Vergleichseinrichtung vorgesehen ist, über die u. a. feststellbar ist, ob eine bestimmte Zeit nach dem Herabsetzen der Hubfrequenz eine Anpassung der tatsächlichen Atemfrequenz des Patienten an die herabgesetzte Hubfrequenz erfolgt ist.

2. Entspannungseinrichtung nach Anspruch 1, dadurch gekennzeichnet, daß die Steuereinrichtung derart ausgebildet ist, daß nach einer Absenkung der Hubfrequenz diese Absenkung dann rückgängig gemacht wird und die Vibrationsbewegung dann wieder mit der ursprünglichen Hubfrequenz erfolgt, wenn nach einer bestimmten Zeit keine Anpassung der Atemfrequenz des Patienten an die abgesenkte Hubfrequenz erfolgt ist, und daß nach einem weiteren Zeitintervall ein erneutes Absenken der Hubfrequenz und ein weiterer Vergleich der tatsächlichen Atemfrequenz mit der neu eingestellten Hubfrequenz erfolgt.

3. Entspannungseinrichtung nach Anspruch 1 oder 2, dadurch gekennzeichnet, daß die Steuereinrichtung dahingehend ausgebildet ist, daß laufend die Atemfrequenz des Patienten ermittelt und mit der tatsächlichen Hubfrequenz verglichen wird, und daß bei einem schnelleren Absinken der tatsächlichen Atemfrequenz des Patienten im Vergleich zu der programmgemäß vorgesehenen stufenweisen Absenkung die Hubfrequenz der Vibrationseinrichtung der schneller als vorgesehen herabgesetzten Atemfrequenz des Patienten angepaßt wird.

4. Entspannungseinrichtung nach einem der Ansprüche 1 bis 3, dadurch gekennzeichnet, daß die Sensoreinrichtung zur Erfassung der Atemfrequenz des Patienten einen in einem Brustgurt (6) vorgesehenen Atemsensor (7) aufweist.

5. Entspannungseinrichtung nach einem der Ansprüche 1 bis 4, dadurch gekennzeichnet, daß
eine Luftbewegungseinrichtung einen Hochdruck-Lüfter (11) aufweist, über den dem aufblasbaren Körper (1) ständig Druckluft zugeführt wird, und daß
die dem aufblasbaren Körper zugeführte Auslaßluftmenge über die Steuereinrichtung (5) steuerbar ist.

6. Entspannungseinrichtung nach Anspruch 5,
dadurch gekennzeichnet, daß
die Steuereinrichtung (5) ein Rohr (14) mit über einen Schieber (16) einstellbaren Luftaustrittsöffnung (15) aufweist, und
der Schieber (16) über einen Stellmotor (18) verstellbar ausgebildet ist.

7. Entspannungseinrichtung nach einem der Ansprüche 1 bis 6, dadurch gekennzeichnet, daß die Steuereinrichtung (5) eine Einstelleinrichtung zur manuellen Eingabe der Atemfrequenz bzw. der Hubfrequenz aufweist.

## Claims

1. A relaxation apparatus comprising a vibrator device and a mattress (1) which is connected thereto and which can be caused to vibrate and which has an inflatable body in which at least the support surface (2) is made from a flexible material, wherein the vibrator device has a control device (5) for periodically varying the internal pressure of the inflatable body, characterised in that there is provided a sensor device (7) for detecting the respiration rate of a patient lying on the support surface, and by means of the control device the upward stroke movement of the support surface can be adapted to the inhalation phase and the downward stroke movement of the support surface can be adapted to the exhalation phase and the post-expiratory phase can be adapted to a stroke movement stoppage, and associated with the control device is a table storage device with a table which contains the average relative duration of the inhalation phase, the exhalation phase and the post-expiratory rest phase at different respiration rates, and the stroke movement frequency is effected according to the stored table values corresponding to the respectively measured respiration rate of the patient or set respiration rate, and associated with the control device is a sequential control device, by the action of which the stroke movement frequency of the vibrator device can be reduced stepwise at given intervals of time, and there is provided a comparison device by which inter alia it can be established whether a given time after the reduction in the stroke movement frequency adaptation of the actual respiration rate of the patient to the reduced stroke movement frequency has occurred.

2. A relaxation apparatus according to claim 1 characterised in that the control device is such that after a reduction in the stroke movement frequency that reduction is then reversed and the vibratory movement is then again effected with the original stroke movement frequency if after a given time no adaptation of the respiration rate of the patient to the reduced stroke movement frequency has occurred, and that after a further time interval a renewed reduction in the stroke movement frequency and a further comparison of the actual respiration rate with the newly set stroke movement frequency are effected.

3. A relaxation apparatus according to claim 1 or claim 2 characterised in that the control device is so designed that the respiration rate of the patient is continuously ascertained and compared to the actual stroke movement frequency, and that in the event of a faster fall in the actual respiration rate of the patient in comparison with the stepwise reduction which is provided in accordance with the program the stroke movement frequency of the vibrator device is adapted to the patient respiration rate which is reduced faster than intended.

4. A relaxation apparatus according to one of claims 1 to 3 characterised in that the sensor device, for detecting the respiration rate of the patient, has a respiration sensor (7) provided in a chest belt (6).

5. A relaxation apparatus according to one of claims 1 to 4 characterised in that an air movement device has a high pressure fan (11) by way of which air under pressure is continuously supplied to the inflatable body (1) and that the amount of exhaust air which is supplied to the inflatable body is controllable by way of the control device (5).

6. A relaxation apparatus according to claim 5 characterised in that the control device (5) has a tube (14) with an air outlet opening (15) which is adjustable by way of a slider (16), and the slider (16) is adapted to be displaceable by way of a control motor (18).

7. A relaxation apparatus according to one of claims 1 to 6 characterised in that the control device (5) has an adjusting device for manual input of the respiration rate and the stroke movement frequency respectively.

## Revendications

1. Dispositif de relaxation avec un dispositif vibratoire et un matelas (1) raccordé à ce dispositif vibratoire et pouvant être soumis aux vibrations, lequel matelas présente un corps gonflable, dont au moins la surface de couchage (2) est composée d'un matériau flexible, le dispositif vibratoire présentant un dispositif de commande (5) prévu pour le changement périodique de la pression intérieure du corps gonflable, **caractérisé en ce qu'**un dispositif à capteur (7) est prévu pour l'enregistrement de la fréquence respiratoire d'un patient allongé sur la surface de couchage, et qu'au moyen du dispositif de commande la course ascendante de la surface de couchage est adaptable à l'inspiration et la course descendante de la surface de couchage est adaptable à l'expiration et la phase post-expiratoire à un arrêt de course, et qu'un dispositif d'enregistrement de tableaux avec un tableau comprenant la durée relative moyenne de l'inspiration, de l'expiration et de la phase de repos post-expiratoire lors de différentes fréquences respiratoires, est attribué au dispositif de commande, et que la fréquence de course est exécutée en fonction des valeurs mémorisées du tableau correspondant à la fréquence respiratoire mesurée sur le patient ou à la fréquence respiratoire réglée, et qu'un dispositif de commande secondaire est attribué au dispositif de commande grâce à l'action duquel la fréquence de course du dispositif vibratoire peut être diminuée graduellement par intervalles de temps définis, et qu'un dispositif de comparaison est prévu à l'aide duquel on peut entre autres constater si, une certaine période de temps après la diminution de la fréquence de course, une adaptation de la fréquence respiratoire effective du patient à la fréquence de course diminuée a eu lieu.

2. Dispositif de relaxation selon la revendication 1, **caractérisé en ce que** le dispositif de commande est formé de telle sorte qu'après une diminution de la fréquence de course, cette diminution est annulée et le mouvement vibratoire a de nouveau lieu à la fréquence de course d'origine, si après une certaine période de temps aucune adaptation de la fréquence respiratoire du patient à la fréquence de course diminuée n'a eu lieu, et en ce qu'après un autre intervalle de temps une nouvelle diminution de la fréquence de course et une autre comparaison de la fréquence respiratoire effective sont réalisées avec la nouvelle fréquence de course réglée.

3. Dispositif de relaxation selon la revendication 1 ou 2, **caractérisé en ce que** le dispositif de commande est formé de sorte que la fréquence respiratoire du patient est détectée en permanence et comparée avec la fréquence de course effective, et en ce qu'en cas de diminution plus rapide de la fréquence respiratoire effective du patient par rapport à la diminution graduelle prévue selon le programme, la fréquence de course du dispositif vibratoire est adaptée à la fréquence respiratoire réglée du patient diminuée plus rapidement que prévu.

4. Dispositif de relaxation selon l'une des revendications 1 à 3, **caractérisé en ce que** le dispositif à capteur pour l'enregistrement de la fréquence respiratoire du patient présente un capteur de respiration (7) prévu dans une ceinture de thorax (6).

5. Dispositif de relaxation selon l'une des revendications 1 à 4, **caractérisé en ce qu'**un dispositif de circulation d'air présente un ventilateur à haute pression (11) à l'aide duquel de l'air comprimé est amené au corps gonflable (1) en permanence et en ce que la quantité d'air de sortie amenée au corps gonflable est réglable au moyen du dispositif de commande (5).

6. Dispositif de relaxation selon la revendication 5, **caractérisé en ce que** le dispositif de commande (5) présente un tuyau (14) avec une ouverture de sortie d'air (15) réglable à l'aide d'un obturateur (16) et que l'obturateur (16) est formé de façon réglable à l'aide d'un servomoteur (18).

7. Dispositif de relaxation selon l'une des revendications 1 à 6, **caractérisé en ce que** le dispositif de commande (5) présente un dispositif de réglage pour l'entrée manuelle de la fréquence respiratoire ou de la fréquence de course.
